# FASCICULE DE BREVET EUROPEEN

(11) **EP 3 302 375 B1**
(45) Date de publication et mention de la délivrance du brevet: **04.11.2020**
(21) Numéro de dépôt: 16730741.2
(22) Date de dépôt: 06.06.2016
(51) Int. Cl.: A61F 5/02, A61F 5/30

(54) **DISPOSITIF D'AIDE POUR AMELIORER LA POSTURE**
HILFE ZUR HALTUNGSVERBESSERUNG
POSTURE IMPROVEMENT AID

(30) Priorité: 08.06.2015 FR 1555213
(43) Date de publication de la demande: 11.04.2018
(73) Titulaire: Percko, 75010 Paris (FR)
(72) Inventeur: PERRAUDEAU, Quentin, 79100 Thouars (FR); UCKO, Alexis, 67000 Strasbourg (FR); LE BORGNE, Pol, 75009 Paris (FR); VIALLON, Hugo, 26000 Valence (FR)
(74) Mandataire: Cabinet Camus Lebkiri
(86) Numéro de dépôt international: PCT/EP2016/062803
(87) Numéro de publication internationale: WO 2016/198358

(56) Documents cités:
- DE-U1-202006 004 993
- JP-A- 2006 291 399
- JP-A- 2013 087 385
- US-A- 5 267 947
- US-A1- 2014 196 190

## Description

### DOMAINE TECHNIQUE DE L'INVENTION

La présente invention concerne un dispositif d'aide pour améliorer la posture d'un utilisateur. L'invention trouve une application particulièrement intéressante dans la correction d'une mauvaise posture et l'optimisation de la respiration.

### ARRIERE PLAN TECHNOLOGIQUE DE L'INVENTION

Aujourd'hui, de nombreuses personnes souffrent de maux de dos qui sont dus notamment à une mauvaise posture aussi bien en position statique que dynamique.

Une mauvaise posture se définit par rapport à la colonne vertébrale, également appelée rachis, vue de profil qui a la forme d'un « S » allongé. La mauvaise posture concerne en particulier les utilisateurs d'ordinateur qui assis en position statique, passent plusieurs heures le dos voûté. Une telle posture peut entraîner des douleurs, des déformations telles qu'une hyperlordose, une hypercyphose ou encore une scoliose et à terme peut être à l'origine de troubles fonctionnels. Par ailleurs, une mauvaise posture peut limiter la quantité d'air inhalée lors de la respiration ce qui peut avoir des effets néfastes sur l'état de santé général.

Il est connu d'utiliser des dispositifs permettant de corriger la mauvaise posture. Par exemple, la demande de brevet WO 2012/168883 décrit un dispositif pour améliorer la posture d'un utilisateur. Le dispositif comprend une ceinture destinée à entourer le thorax d'un utilisateur ainsi que deux sangles dont les extrémités sont également destinées à entourer le thorax d'un utilisateur lorsqu'elles sont fixées l'une sur l'autre, lesdites extrémités se superposant à la ceinture. Par ailleurs, chaque sangle comprend une partie avant et une partie arrière adaptées pour être positionnées respectivement à l'avant et à l'arrière de l'épaule, les parties arrière des sangles étant croisées. De plus, chaque sangle comprend une bande, ladite bande étant reliée à la partie avant de la sangle et à la partie arrière de la sangle de sorte à passer sous chaque aisselle de l'utilisateur.

Un inconvénient d'un tel dispositif est que la ceinture ainsi que les extrémités des sangles entourant le thorax peuvent limiter son ouverture et ainsi empêcher l'utilisateur de respirer correctement. De plus, le dispositif ne permet pas de corriger les mauvaises postures au niveau de la partie inférieure du dos. Enfin, l'utilisateur peut avoir des difficultés à réaliser certains mouvements.

D'autres dispositifs d'aide pour améliorer la posture d'un utilisateur sont divulgués, notamment, dans DE 20 2006 004993 U1, JP 2013 087385 A et JP 2006 291399 A.

### DESCRIPTION GENERALE DE L'INVENTION

Dans ce contexte, l'invention vise à fournir une solution permettant d'adopter et de maintenir une bonne posture sur la partie inférieure du dos d'un utilisateur tout en laissant l'utilisateur respirer correctement et être libre de ses mouvements.

L'invention concerne un dispositif d'aide pour améliorer la posture d'un utilisateur tel que défini dans la revendication indépendante 1. Différents modes de réalisation préférentiels sont définis dans les revendications dépendantes.

Par « ceinture », on entend une bande de matière, pouvant être extensible, destinée à entourer horizontalement une partie du corps d'un utilisateur. On appelle « plan horizontal » un plan parallèle au plan transversal du corps.

Par « tenseur », on entend une bande de matière, pouvant être extensible, destinée à être positionnée verticalement ou obliquement sur une partie du corps d'un utilisateur. On appelle « plan vertical » et « plan oblique », un plan respectivement perpendiculaire et oblique par rapport au plan transversal du corps. Les tenseurs sont positionnés par paire sur le corps d'un utilisateur, chaque tenseur d'une paire étant symétrique du second tenseur par rapport au plan médian d'un utilisateur.

Le dispositif selon l'invention répond aux contraintes préalablement citées.

La coopération de la ceinture avec les tenseurs latéraux par l'intermédiaire de la liaison élastique permet au dispositif d'agir sur une zone spécifique du dos appelée zone de stimulation. La zone de stimulation, positionnée entre la troisième vertèbre lombaire et la cinquième vertèbre lombaire de l'utilisateur, permet, en cas de mauvaise posture, une stimulation optimale du dos d'un utilisateur. L'élément positionné sur la zone de stimulation présente une forme et des dimensions adaptées pour provoquer un contact entre l'élément et le corps d'un utilisateur lorsque la zone de stimulation est mise sous tension. En effet, une mauvaise posture consiste, par exemple, à maintenir un dos arrondi qui entraine une déformation de la lordose lombaire physiologique. La lordose lombaire étant localisée entre la troisième vertèbre lombaire et la cinquième vertèbre lombaire, une mise en tension de la zone de stimulation permet de stimuler directement la zone dont la courbure physiologique doit être rétablie. Ainsi, une utilisation régulière d'un tel dispositif permet à un utilisateur de combattre et de prévenir d'une mauvaise posture pouvant être à l'origine de maux de dos. Par ailleurs, le dispositif permet, grâce à la ceinture, de maintenir le bassin dans une position optimale. En outre, le dispositif ne bloque pas l'ouverture du thorax de l'utilisateur, il peut ainsi respirer correctement tout en restant libre de ses mouvements.

Le dispositif selon l'invention peut également présenter une ou plusieurs des caractéristiques ci-dessous, considérées individuellement ou selon toutes les combinaisons techniquement possibles.

Selon un mode de réalisation non limitatif, la liaison élastique est construite et agencée de sorte que lorsque la partie arrière de la ceinture est positionnée sur les épines iliaques postéro-supérieures de l'utilisateur, la zone de stimulation est positionnée sur la cinquième vertèbre lombaire.

Selon un mode de réalisation non limitatif, le dispositif comporte des moyens d'ouverture du thorax comprenant une partie avant et une partie arrière fixées au premier tenseur latéral et au un second tenseur latéral, la jonction entre la partie avant et la partie arrière étant construite et agencé pour se positionner sur les épaules d'un utilisateur.

Selon un mode de réalisation non limitatif, la seconde extrémité du premier tenseur latéral et la seconde extrémité du second tenseur latéral sont fixées à la partie arrière des moyens d'ouverture du thorax, les tenseurs formant chacun un angle compris dans l'intervalle de valeurs [65°, 73°], préférentiellement 71.2° avec un axe sensiblement perpendiculaire à l'axe longitudinal de la ceinture et passant par la zone de stimulation.

Selon un mode de réalisation non limitatif, la partie avant et la partie arrière des moyens d'ouverture du thorax forment des bretelles.

Selon un mode de réalisation non limitatif, la partie avant et la partie arrière des moyens d'ouverture du thorax sont réalisées dans un matériau présentant une élasticité plus faible selon un axe vertical que selon un axe horizontal, ledit axe vertical étant sensiblement perpendiculaire à l'axe longitudinal de la ceinture.

Selon un mode de réalisation non limitatif, un patronage des moyens d'ouvertures du thorax forme des moyens de maintien du thorax, ledit patronage étant ajusté selon une direction sensiblement parallèle à l'axe longitudinal de la ceinture.

Selon un mode de réalisation non limitatif, le dispositif comporte :
▪ un premier tenseur axial comprenant une première extrémité fixée à la zone de stimulation et une seconde extrémité fixée aux moyens d'ouverture du thorax,
▪ un second tenseur axial comprenant une première extrémité fixée à la zone de stimulation et une seconde extrémité fixée aux moyens d'ouverture du thorax.

Selon un mode de réalisation non limitatif, la seconde extrémité du premier tenseur axial et la seconde extrémité du second tenseur axial sont fixées à la partie arrière des moyens d'ouverture du thorax, les tenseurs formant chacun un angle compris dans l'intervalle de valeurs [15°, 21°], préférentiellement 18° avec un axe sensiblement perpendiculaire à l'axe longitudinal de la ceinture et passant par la zone de stimulation.

Selon un mode de réalisation non limitatif, l'élément 9 positionné sur la zone de stimulation est fixé à la zone de stimulation du premier tenseur latéral et du second tenseur latéral par intégration dudit élément dans une poche, ladite poche étant fixée à la zone de stimulation.

Selon un mode de réalisation non limitatif, la ceinture comporte des premiers moyens de maintien en position du dispositif.

Selon un mode de réalisation non limitatif, les premiers moyens de maintien sont formés par au moins un premier élément antidérapant fixé sur la partie avant et sur la partie arrière de la ceinture de sorte que lorsque l'utilisateur porte le dispositif, le premier élément antidérapant soit en contact avec le bassin de l'utilisateur.

Selon un mode de réalisation non limitatif, le dispositif comporte des seconds moyens de maintien en position du dispositif.

Selon un mode de réalisation non limitatif, les seconds moyens de maintien sont formés par un second élément antidérapant et un troisième élément antidérapant maintien construits et agencés pour être positionnés respectivement sur un premier flanc et un second flanc de l'utilisateur.

Selon un mode de réalisation non limitatif, la fixation des tenseurs sur les moyens d'ouvertures du thorax est réalisée par contre-collage desdits tenseurs sur lesdits moyens d'ouverture du thorax.

### BREVE DESCRIPTION DES FIGURES

Les figures ne sont présentées qu'à titre indicatif et nullement limitatif.
La figure 1 représente schématiquement une vue de profil d'une colonne vertébrale présentant des courbures physiologiques.
La figure 2 représente schématiquement une vue avant du dispositif selon un mode de réalisation de l'invention.
La figure 3 représente schématiquement une vue arrière du dispositif selon le mode de réalisation présenté à la figure 2.
La figure 4 représente schématiquement une vue latérale du positionnement de l'élément du dispositif par rapport à la colonne vertébrale d'un utilisateur selon un mode de réalisation de l'invention.
La figure 5 représente schématiquement une vue avant du dispositif, selon le mode de réalisation présenté à la figure 2, lorsqu'il est porté par un utilisateur.
La figure 6 représente schématiquement une vue arrière du dispositif, selon le monde de réalisation présenté à la figure 3, lorsqu'il est porté par un utilisateur.

### DESCRIPTION DETAILLEE DE MODES DE REALISATION DE L'INVENTION

Sauf précision contraire, un même élément apparaissant sur des figures différentes présente une référence unique.

Par ailleurs, on nommera, dans la suite de la description, « axe Y », un axe appartenant au plan médian et perpendiculaire au plan transversal d'un corps humain. On entend également par « axe Y » l'axe de symétrie du dispositif de l'invention.

L'invention concerne un dispositif DISP d'aide pour améliorer la posture d'un utilisateur UTL. Pour ce faire, le dispositif agit sur plusieurs points de la colonne vertébrale CV.

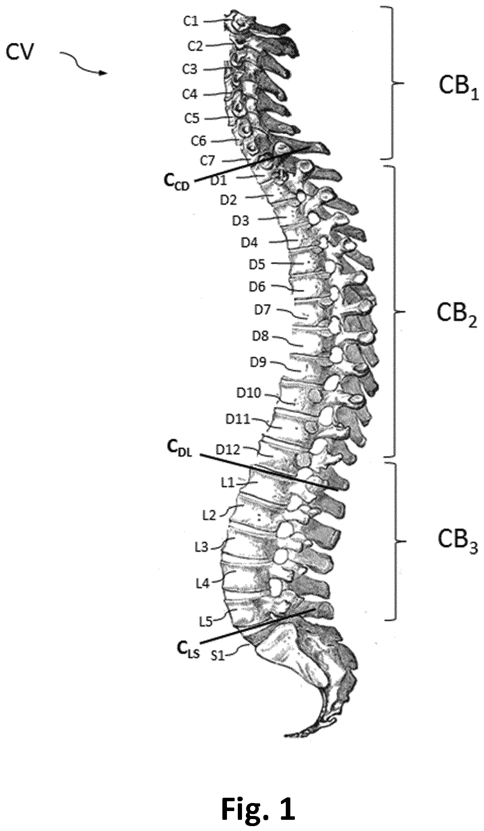

Pour rappel, la colonne vertébrale CV d'un corps humain représentée sur la figure 1 comprend sept vertèbres cervicales C1 à C7, douze vertèbres dorsales D1 à D12 et cinq vertèbres lombaires L1 à L5.

La figure 1 représente les courbures dites physiologiques qui conditionnent l'équilibre de la colonne vertébrale CV. Ainsi, la colonne vertébrale CV comprend trois courbures : une première dans la section cervicale CB₁ dite lordose cervicale, une deuxième dans la section dorsale CB₂ dite cyphose dorsale et une troisième dans la section lombaire CB₃ dite lordose lombaire. L'alternance des courbures se manifeste au niveau des différentes charnières de la colonne vertébrale CV : la charnière cervico-dorsale C_{CD} (C7-D1), la charnière dorso-lombaire C_{DL} (D12-L1) et la charnière lombo-sacrée C_{LS} (L5-S1).

En cas de mauvaise posture, la colonne vertébrale CV est déformée de sorte qu'elle présente des courbures différentes des courbures CB₁, CB₂ et CB₃ présentées sur la figure 1. Par exemple, une hypercyphose est une accentuation de la courbure CB₁ vers l'arrière. Une hyperlordose est une accentuation vers l'avant de la courbure CB₂.

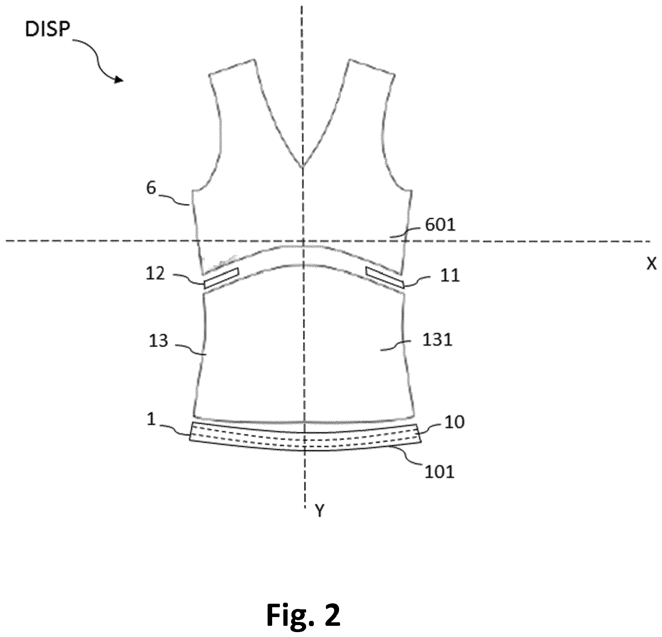

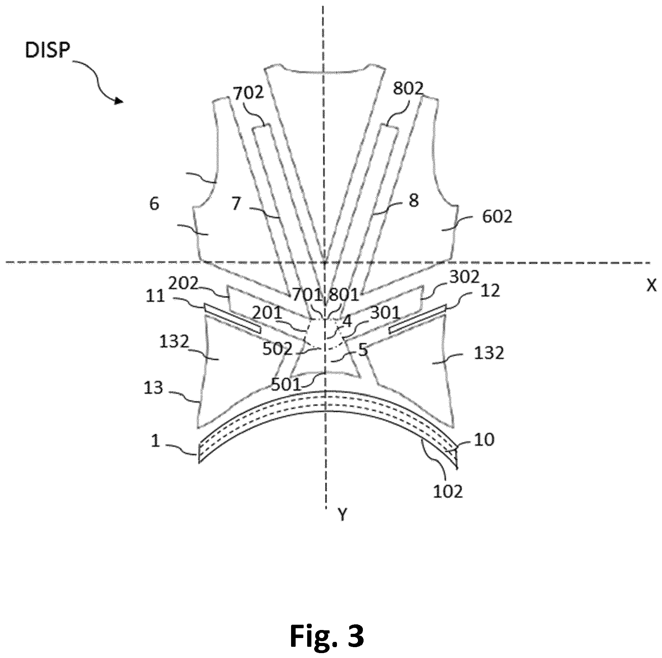

Les figures 2 et 3 représentent respectivement une vue avant et une vue arrière des différents éléments que comporte le dispositif DISP de l'invention selon un mode de réalisation.

En référence aux figures 2 et 3, le dispositif DISP comporte :
- une ceinture 1,
- des premiers moyens de maintien 10,
- une liaison élastique 5,
- un premier tenseur latéral 2 et un second tenseur latéral 3,
- des seconds moyens de maintien (11, 12),
- un support de fixation 13,
- des moyens d'ouverture du thorax 6,
- un premier tenseur axial 7 et un second tenseur axial 8.

On note que les figures 2 et 3 représentent des vues éclatées du dispositif DISP selon l'invention. Selon un mode de réalisation, chaque élément précité 1 à 13 est fixé à au moins un desdits éléments 1 à 13 de sorte que le dispositif DISP forme un unique élément tel qu'un vêtement.

La ceinture 1 comporte une partie avant 101, représentée sur la figure 2, et une partie arrière 102, représentée sur la figure 3. La partie avant 101 de la ceinture 1 est destinée à être positionnée sur la face avant d'un utilisateur UTL et la partie arrière 102, sur la face arrière de l'utilisateur UTL. Plus précisément, la ceinture 1 est destinée à entourer le bassin de l'utilisateur UTL en recouvrant notamment ses épines iliaques postéro-supérieures.

En effet, la ceinture 1 présente une longueur et une largeur dimensionnées pour entourer correctement l'ensemble du bassin d'un utilisateur UTL tout en exerçant une tension sur ledit bassin lorsque l'utilisateur UTL adopte une mauvaise posture. La longueur et la largeur de la ceinture 1 sont également choisies de sorte à ne pas gêner la respiration de l'utilisateur UTL. Selon un mode de réalisation, pour un dispositif DISP adapté à une taille large « L », la ceinture 1 présente une longueur de 962 mm et une largeur de 116 mm. En outre, l'épaisseur de la ceinture 1 est choisie de sorte à minimiser la visibilité du dispositif DISP lorsqu'un utilisateur UTL porte ledit dispositif DISP sous un vêtement. Selon un mode de réalisation, l'épaisseur de la ceinture 1 est de 0.5 mm. De plus, la ceinture 1 est déformable élastiquement de sorte à garantir à l'utilisateur UTL une liberté de mouvements et une respiration optimale. Selon un mode de réalisation, la ceinture 1 est composée à 39% d'élasthanne et à 61% de polyamide.

Selon un mode de réalisation, la ceinture 1 comprend des moyens de réglage de la longueur (non représentés sur les figures 2 et 3), appelés moyens de serrage. Les moyens de serrage permettent au dispositif DISP de s'adapter à la morphologie d'un utilisateur UTL. Selon un mode de réalisation, les moyens de serrage sont formés par un système de scratch. Selon un autre mode de réalisation, les moyens de serrage sont formés par un nœud. Selon un autre mode de réalisation, les moyens de serrage sont formés par une boucle. Selon un autre mode de réalisation, les moyens de serrage sont formés par un système de crochets. Selon un autre mode de réalisation, des moyens de serrage consistent à utiliser les propriétés élastiques de la ceinture 1. En effet, après étirement, un élément élastique essaye, par sa force de rappel, de reprendre sa position d'équilibre. Ainsi, une ceinture élastique épouse la forme de la partie du corps sur laquelle elle est positionnée en appliquant une force de rappel élastique sur ladite partie.

Les premiers moyens de maintien en position 10 sont fixés sur la ceinture 1 de sorte à maintenir un positionnement correct de la ceinture 1 sur le corps d'un utilisateur UTL lorsqu'il porte le dispositif DISP.

En effet, les premiers moyens de maintien 10 sont formés par un premier élément antidérapant 10. La fixation du premier élément antidérapant 10 est réalisée sur la face de la ceinture 1 qui est destinée à être en contact avec le bassin de l'utilisateur UTL lorsqu'il porte le dispositif DISP. En effet, le premier élément antidérapant 10 empêche la ceinture 1 de se déplacer sur le corps de l'utilisateur UTL lorsqu'il est en mouvement par exemple. Pour rappel, la ceinture 1 doit être maintenue fixe sur les épines iliaques postéro-supérieures de l'utilisateur UTL. Selon un mode de réalisation, le premier élément antidérapant 10 est une bande de silicone.

La liaison élastique 5 comporte une première extrémité 501 et une seconde extrémité 502 destinées à être positionnées sur la face arrière de l'utilisateur UTL. La première extrémité 501 de la liaison élastique 5 est fixée sur la partie arrière 102 de la ceinture 1. Plus précisément, la première extrémité 501 est fixée sur la partie centrale de la partie arrière 102 de la ceinture 1. La seconde extrémité 502 de la liaison élastique 5 est fixée sur une zone de stimulation 4.

La liaison élastique 5 présente une longueur et une largeur dimensionnées de sorte que la seconde extrémité 502 de la liaison élastique 5 soit sensiblement proche de la région comprise entre la troisième vertèbre lombaire et la cinquième vertèbre lombaire de l'utilisateur UTL lorsqu'il porte le dispositif DISP. La liaison élastique 5 est déformable élastiquement de sorte que la seconde extrémité 502 peut être positionnée sensiblement proche de ladite région par déformation élastique de la liaison élastique 5. Selon un mode de réalisation, pour un dispositif DISP adapté à une taille large « L », la liaison élastique 5 présente une longueur 160 mm selon un axe X perpendiculaire à l'axe Y. Par ailleurs, selon un mode de réalisation, la liaison élastique 5 présente une largeur de 77 mm selon l'axe Y. En outre, l'épaisseur de la liaison élastique 5 est choisie de sorte à minimiser la visibilité du dispositif DISP lorsqu'un utilisateur UTL porte ledit dispositif DISP sous un vêtement. Ainsi, selon un mode de réalisation, l'épaisseur de la liaison élastique 5 est de 0.5 mm. Selon un mode de réalisation, la liaison élastique 5 est composée à 39% d'élasthanne et à 61% de polyamide.

Le premier tenseur latéral 2 comporte une première extrémité 201 et une seconde extrémité 202. Le second tenseur latéral 3 comporte une première extrémité 301 et une seconde extrémité 302. Le premier tenseur latéral 2 et le second tenseur latéral 3 sont destinés à être positionnés sur la face arrière de l'utilisateur UTL. La première extrémité 201 du premier tenseur latéral 2 et la première extrémité 301 du second tenseur latéral 3 sont fixées à la seconde extrémité 502 de la liaison élastique 5. La seconde extrémité 202 du premier tenseur latéral 2 et la seconde extrémité 302 du second tenseur latéral 3 sont fixés aux moyens d'ouverture du thorax 6.

Par ailleurs, la première extrémité 201 du premier tenseur latéral 2 et la première extrémité 301 du second tenseur latéral 3 se rejoignent au niveau d'une zone, appelée zone de stimulation 4. En effet, lorsqu'un utilisateur UTL porte le dispositif DISP, la zone de stimulation 4 est positionnée sur la région comprise entre la troisième vertèbre lombaire et la cinquième vertèbre lombaire, préférentiellement la cinquième vertèbre lombaire. Ainsi, un tel positionnement de la zone de stimulation 4 permet, en cas de mauvaise posture de l'utilisateur UTL, aux tenseurs latéraux 2 et 3 d'exercer une tension sur ladite région. Par ailleurs, selon un mode de réalisation, un élément 9 est positionné sur la zone de stimulation 4 pour optimiser la stimulation sur la région. Ainsi, la mise en tension de la zone de stimulation 4 stimule l'élément 9 qui vient en appui sur la région, de l'utilisateur UTL, comprise entre la troisième vertèbre lombaire et la cinquième vertèbre lombaire, préférentiellement la cinquième vertèbre lombaire.

Le premier tenseur latéral 2 et le second tenseur latéral 3 présentent une longueur, une largeur, ainsi qu'un angle formé par chaque tenseur latéral 2 et 3 avec l'axe Y permettant d'optimiser l'ouverture du thorax d'un utilisateur UTL lorsqu'il porte le dispositif DISP. En outre, le premier tenseur latéral 2 et le second tenseur latéral 3 sont symétriques par rapport à l'axe Y afin qu'ils appliquent la même tension de part et d'autre dudit axe Y. Selon un mode de réalisation, le premier tenseur latéral 2 et le second tenseur latéral 3 présentent une longueur de 232 mm, une largeur de 56 mm et un angle formé par chaque tenseur latéral 2 et 3 avec l'axe Y de 71,2°. En outre, l'épaisseur du premier tenseur latéral 2 et du second tenseur latéral 3 est choisie de sorte à minimiser la visibilité du dispositif DISP lorsqu'un utilisateur UTL porte ledit dispositif DISP sous un vêtement. Ainsi, selon un mode de réalisation, l'épaisseur du premier tenseur latéral 2 et du second tenseur latéral 3 est de 0.5 mm. Par ailleurs, selon un mode de réalisation, les tenseurs latéraux 2 et 3 sont déformables élastiquement. Selon un mode de réalisation, les tenseurs latéraux 2 et 3 sont composés à 39% d'élasthanne et à 61% de polyamide.

Le support de fixation 13 comporte une partie avant 131 représentée sur la figure 2 et une partie arrière 132 représentée sur la figure 3. La partie avant 131 est destinée à se positionner sur la face avant d'un utilisateur UTL, la partie arrière 132 sur la face arrière de l'utilisateur UTL. La ceinture 1, la liaison élastique 5 ainsi que les tenseurs latéraux 2 et 3 sont fixés sur le support de fixation 13. Le support de fixation 13 permet aux secondes extrémités 202 et 302 des tenseurs latéraux 2 et 3 d'avoir un point de fixation sur le dispositif DISP.

Selon un mode de réalisation, le support de fixation 13 est un matériau textile. Ledit matériau textile permet au dispositif DISP de se présenter sous la forme d'un vêtement et ainsi d'être plus facile à porter par un utilisateur UTL. Selon un mode de réalisation, le textile du support de fixation 13 est composé 85% de polyester et à 15% d'élasthanne. En outre, l'épaisseur du support de fixation 13 est choisie de sorte à minimiser la visibilité du dispositif DISP lorsqu'un utilisateur UTL porte ledit dispositif DISP sous un vêtement. Ainsi, selon un mode de réalisation, l'épaisseur le support de fixation 13 est de 0.4 mm.

Les seconds moyens de maintien en position 11 et 12 du dispositif DISP sont fixés sur la partie avant 131 et sur la partie arrière 132 du support de fixation 13. Sur la partie avant 131 du support de fixation 13, les seconds moyens de maintien en position 11 et 12 sont positionnés sensiblement proche de la jonction entre ladite partie avant 131 et les moyens d'ouverture du thorax 6. Sur la partie arrière 132 du support de fixation 13, les seconds moyens de maintien en position 11 et 12 sont positionnés en-dessous des tenseurs latéraux 2 et 3. Les seconds moyens de maintien en position 11 et 12 sont formés par un second élément antidérapant 11 et un troisième élément antidérapant 12 symétriques par rapport à l'axe Y. Le second élément antidérapant 11 et le troisième élément antidérapant 12 sont destinés à se positionner sur le corps de l'utilisateur UTL respectivement sur un premier flanc et un second flanc de l'utilisateur UTL.

Le second élément antidérapant 11 et le troisième élément antidérapant 12 permettent aux tenseurs latéraux 2 et 3 et plus précisément la zone de stimulation 4 de maintenir une position optimale. Pour rappel, la zone de stimulation 4 doit être maintenue fixe sur la région comprise entre la troisième vertèbre lombaire et la cinquième vertèbre lombaire de l'utilisateur UTL. Le second élément antidérapant 11 et le troisième élément antidérapant 12 permettent ainsi d'éviter un déplacement des tenseurs latéraux 2 et 3 sur le corps de l'utilisateur UTL lorsqu'il est en mouvement par exemple. Selon un mode de réalisation, le second élément antidérapant 11 et le troisième élément antidérapant 12 sont des bandes de silicone.

Les moyens d'ouverture du thorax 6 comportent une partie avant 601 et une partie arrière 602, la partie avant 601 étant destinée à se positionner sur la face avant d'un utilisateur UTL, la partie arrière 602 sur la face arrière de l'utilisateur UTL. La partie avant 601 des moyens d'ouverture du thorax 6 est fixée à la partie avant 131 du support de fixation 15. La partie arrière 602 des moyens d'ouverture du thorax 6 est fixée aux tenseurs latéraux 2 et 3. Par ailleurs, les moyens d'ouverture du thorax 6 sont destinés à encapsuler le thorax d'un utilisateur UTL lorsqu'il porte le dispositif DISP. Les moyens d'ouvertures du thorax 6 recouvrent la partie du corps de l'utilisateur UTL allant de la zone située au-dessus des côtes flottantes jusqu'aux épaules de sorte à former un vêtement.

Par ailleurs, les moyens d'ouverture du thorax 6 sont réalisés dans un matériau présentant une élasticité plus faible selon un axe vertical que selon un axe horizontal, ledit axe vertical étant sensiblement perpendiculaire à l'axe longitudinal de la ceinture 1. En effet, l'utilisation d'un tel matériau incite l'utilisateur UTL à optimiser l'ouverture de son thorax. En effet, l'élasticité du matériau étant plus faible selon l'axe vertical, l'utilisateur UTL est stimulé selon l'axe vertical s'il adopte une mauvaise posture et l'incite à rétablir une courbure physiologique permettant une ouverture optimale du thorax. Par ailleurs, l'élasticité du matériau étant plus grande selon l'axe horizontal, l'utilisateur UTL n'éprouve pas de difficultés à respirer. Selon un mode de réalisation, les moyens d'ouverture du thorax 6 sont réalisés dans un matériau composé à 100% de polyester. En outre, l'épaisseur des moyens d'ouverture du thorax 6 est choisie de sorte à minimiser la visibilité du dispositif DISP lorsqu'un utilisateur UTL porte ledit dispositif DISP sous un vêtement. Ainsi, selon un mode de réalisation, l'épaisseur des moyens d'ouverture du thorax 6 est de 0.4 mm.

Selon un mode de réalisation, un patronage spécifique des moyens d'ouverture du thorax 6 forme des moyens de maintien du thorax. En effet, le patronage des moyens d'ouverture du thorax 6 est réalisé de sorte que lorsqu'un utilisateur UTL porte le dispositif DISP, les moyens d'ouverture du thorax 6 sont ajustés sur l'utilisateur UTL. Ainsi, un tel patronage s'apparente à une infinité de ceintures encapsulant horizontalement le thorax de l'utilisateur UTL. Les moyens d'ouverture du thorax 6 ne couvrant pas les côtes flottantes, l'utilisateur UTL reste libre de ses mouvements lorsqu'il respire.

Dans un autre mode de réalisation, les moyens d'ouverture du thorax 6 forment des bretelles. Chaque bretelle est alors fixée à la partie avant 131 du support de fixation 13 et au premier tenseur latéral 2 ou au second tenseur latéral 3. Par « bretelle », on entend une bande de matière, pouvant être extensible, destinée à être positionnée verticalement de part et d'autre de l'épaule d'un utilisateur UTL, une partie de ladite bretelle passant au-dessus de l'épaule de sorte à l'entourer. On appelle « plan vertical », un plan perpendiculaire par rapport au plan transversal du corps. Les bretelles sont positionnées par paires sur les épaules d'un utilisateur UTL, chaque bretelle étant symétrique de la seconde bretelle par rapport au plan médian de l'utilisateur UTL.

Le premier tenseur axial 7 comporte une première extrémité 701 fixée à la zone de stimulation 4 et une seconde extrémité 702 fixée à la partie arrière 602 des moyens d'ouvertures du thorax 6. Le second tenseur axial 8 comporte une première extrémité 801 fixée à la zone de stimulation 4 et une seconde extrémité 802 fixée à la partie arrière 602 des moyens d'ouvertures du thorax 6. Les tenseurs axiaux 7 et 8 exercent une tension sur le corps d'un utilisateur UTL en cas de mauvaise posture.

Par ailleurs, le premier tenseur axial et le second tenseur axial 8 présentent une longueur, une largeur, ainsi qu'un angle formé par chaque tenseur axial 7 et 8 avec l'axe Y permettant une ouverture optimale du thorax d'un utilisateur UTL lorsqu'il porte le dispositif DISP. En outre, le premier tenseur axial 7 et le second tenseur axial 8 sont symétriques par rapport à l'axe Y afin qu'ils appliquent la même tension de part et d'autre dudit axe Y. Selon un mode de réalisation, le premier tenseur axial 7 et le second tenseur axial 8 présentent une longueur de 482 mm, une largeur de 40 mm et un angle avec l'axe médian de l'utilisateur UTL de 18°. En outre, l'épaisseur du premier tenseur axial 7 et du second tenseur axial 8 est choisie de sorte à minimiser la visibilité du dispositif DISP lorsqu'un utilisateur UTL porte ledit dispositif DISP sous un habit. Ainsi, selon un mode de réalisation, l'épaisseur du premier tenseur axial 7 et du second tenseur axial 8 est de 0.5 mm. Par ailleurs, le premier tenseur axial 7 et le second tenseur axial 8 sont symétriques par rapport à l'axe Y de sorte à appliquer la même tension de part et d'autre dudit axe Y. Selon un mode de réalisation, les tenseurs axiaux 7 et 8 sont déformables élastiquement. Selon un mode de réalisation, les tenseurs axiaux 7 et 8 sont composés à 39% d'élasthanne et à 61% de polyamide.

En outre, les moyens d'ouvertures du thorax 6 permettent aux secondes extrémités 702 et 802 des tenseurs axiaux 7 et 8 d'avoir un point de fixation autre que la zone de stimulation 6 sur le dispositif DISP. Selon un mode de réalisation, les moyens d'ouverture du thorax 6 sont formés par un matériau textile.

Selon un mode de réalisation, les éléments précités 1, 2, 3, 5, 6, 7, 8 à 13 sont assemblés de sorte à former un vêtement pouvant être porté sous un autre vêtement tel qu'une chemise, un tee-shirt etc. L'assemblage des tenseurs 2, 3, 7 et 8 et de la ceinture 1, sur le support de fixation 13 et sur les moyens d'ouverture du thorax 6 permet de répartir les efforts desdits tenseurs 2, 3, 7 et 8 et de la ceinture 1 sur l'ensemble du dos de l'utilisateur UTL.

Selon un mode de réalisation, chaque tenseur 2, 3, 7 et 8 est fixé au support de fixation 13 et/ou aux moyens d'ouverture du thorax 6 par contre-collage. En effet, une fixation par couture génère un relief dans le dispositif DISP qui peut provoquer une gêne à l'utilisateur UTL. En outre, selon un mode de réalisation, la fixation de la partie avant 131 avec la partie arrière 132 du support de fixation 13 est également réalisée par contre-collage.

Selon un mode de réalisation, les tenseurs 2, 3, 7 et 8 et la liaison élastique 5 sont fixés entre eux par couture au niveau de leurs extrémités.

Par ailleurs, selon un mode de réalisation, les tenseurs 2, 3, 7 et 8 présentent une forme arquée. En effet, un tenseur ayant une forme arquée suit le muscle de manière plus précise. Ainsi, les forces exercées agissent directement sur le muscle concerné. Selon un autre mode de réalisation, les tenseurs 2, 3, 7 et 8 sont droits. En effet, lorsque le tenseur est droit, l'activation des forces est plus rapide. Selon un autre mode de réalisation, les tenseurs 2 et 3 présentent une forme arquée et les tenseurs 7 et 8 sont droits ou inversement.

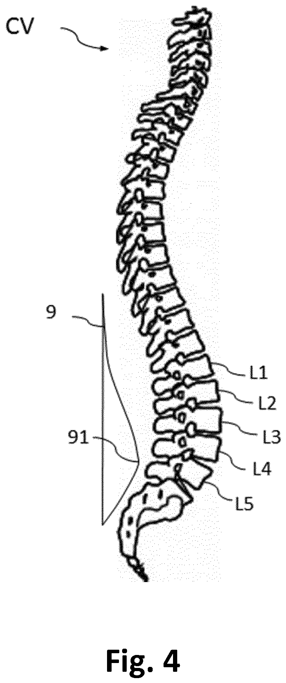

La figure 4 représente une vue latérale du positionnement de l' élément 9 du dispositif DISP par rapport à la colonne vertébrale CV d'un utilisateur UTL lorsqu'il porte le dispositif DISP. L'élément 9 est positionné sur la région comprise entre la troisième vertèbre lombaire L3 et la cinquième vertèbre lombaire L5 de sorte à stimuler ladite région en cas de mauvaise posture de l'utilisateur UTL. La région comprise entre la troisième vertèbre lombaire L3 et la cinquième vertèbre lombaire L5 correspond à la région de la colonne vertébrale CV comprenant la lordose lombaire. Selon l'invention, en référence à la figure 4, un sommet 91 de l'élément 9 est positionné sur la cinquième vertèbre lombaire L5 lorsque le dispositif DISP est porté par l'utilisateur UTL. Ainsi, lorsque la zone de stimulation 4 est mise sous tension, ladite zone de stimulation 4 exerce une tension sur l'élément 9 qui appuie sur le creux de la lordose lombaire et incite ainsi l'utilisateur UTL à se repositionner correctement.

En outre, l'élément 9 présente une longueur, une largeur et une épaisseur adaptées pour une stimulation optimale de la région comprise entre la troisième vertèbre lombaire L3 et la cinquième vertèbre lombaire L5. Ainsi, selon un mode de réalisation, l'élément 9 présente une largeur de 50 mm, une longueur de 183 mm et une épaisseur de 18 mm. L'élément 9 est fixé à la zone de stimulation 4 par intégration dudit élément 9 dans une poche fixée à la zone de stimulation 4. Selon un mode de réalisation, l'élément 9 est une mousse.

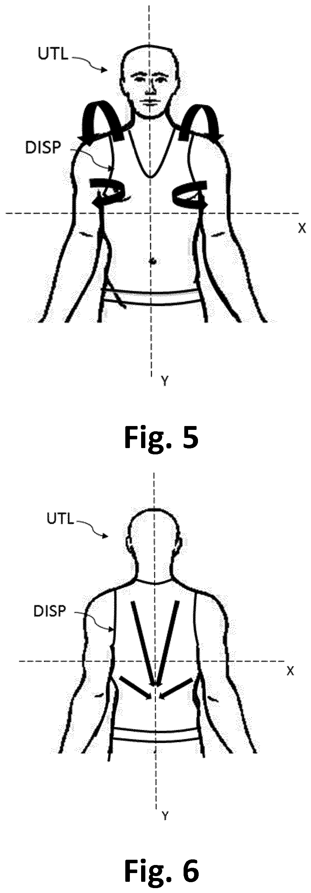

Les figures 5 et 6 représentent respectivement une vue avant et une vue arrière du dispositif DISP lorsqu'il est porté par un utilisateur UTL.

Dans ce mode de réalisation, l'utilisateur UTL porte le dispositif DISP selon le mode de réalisation présenté sur les figures 2 et 3. Ainsi, le dispositif DISP comporte la ceinture 1, le support de fixation 13, le premier tenseur latéral 2, le second tenseur latéral 3, les premiers et les seconds moyens de maintien en position 10, 11 et 12, le premier tenseur axial 7, le second tenseur axial 8, les moyens d'ouverture du thorax 6 et l'élément 9. Les éléments précités coopèrent ensemble pour corriger une mauvaise posture. Le rétablissement et le maintien d'une bonne posture permettent de prévenir les maux de dos. Par ailleurs, la correction d'une mauvaise posture permet d'assurer une ouverture optimale du thorax et ainsi d'améliorer la respiration d'un utilisateur UTL.

Une mauvaise posture consiste par exemple à maintenir un dos voûté pendant un certain temps. Les épaules sont alors enroulées vers l'avant et l'ensemble du dos est également incliné vers l'avant. Cette position peut être à l'origine de douleurs dorsales dus à une diminution de la lordose lombaire, à une augmentation de la cyphose dorsale et à une augmentation de la lordose cervicale.

Ainsi, lorsqu'un utilisateur UTL porte le dispositif DISP et que celui-ci a le dos voûté, la partie arrière des moyens d'ouverture du thorax 6 est soumise à une tension dirigée vers l'avant de l'utilisateur UTL car les épaules sont inclinées vers l'avant. Par ailleurs, l'ensemble du dos, plus particulièrement la partie supérieure du dos subit également une déformation plus ou moins importante selon la distance qui la sépare des épaules. Ainsi, les tenseurs axiaux 7 et 8 et les tenseurs latéraux 2 et 3, la liaison élastique 5 ainsi que la partie arrière 102 de la ceinture 1 sont également soumis à une tension dirigée vers l'avant de l'utilisateur UTL.

Ainsi, chaque élément 2, 3, 5, 6, 7 et 8 tentant de reprendre sa position d'équilibre applique une tension dirigée vers l'arrière de l'utilisateur UTL sur la zone sur laquelle ledit élément est positionné. Les tensions appliquées par lesdits éléments 2, 3, 5, 6, 7 et 8 stimulent alors l'utilisateur UTL sur plusieurs zones ce qui provoque une gêne à l'utilisateur UTL et l'informe ainsi de son mauvais positionnement.

Lorsque le dispositif DISP est porté par un utilisateur UTL dont le dos est voûté, une stimulation au niveau de ses épaules est réalisée par les moyens d'ouverture du thorax 6. De plus, l'ensemble du thorax est stimulé par lesdits moyens d'ouverture du thorax 6, les tenseurs latéraux 2 et 3, les tenseurs axiaux 7 et 8 ainsi que l'élément 9.

En effet, en cas de mauvaise posture, les moyens d'ouverture du thorax 6, qui présentent une élasticité faible dans un sens vertical induisent, une rotation des épaules selon l'axe X vers la face arrière de l'utilisateur UTL telle que représentée sur la figure 5. Ainsi, les moyens d'ouverture du thorax 6 permettent de redresser les épaules et de maintenir le dos de l'utilisateur UTL droit afin de maintenir la lordose cervicale et la cyphose dorsale physiologiques. De plus, les moyens d'ouvertures du thorax 6 encapsulent le thorax horizontalement de sorte à optimiser son ouverture.

Les tenseurs latéraux 2 et 3 sont également soumis à une tension lorsque l'utilisateur UTL adopte une mauvaise posture. Plus précisément, la mise en tension des tenseurs latéraux 2 et 3 incite, par stimulation, l'utilisateur UTL à réaliser une rotation de la partie latérale gauche et une rotation de la partie latérale droite du thorax telles que représentées sur la figure 5. Lesdites rotations sont réalisées suivant l'axe Y et sont dirigées vers la face arrière de l'utilisateur UTL. Ladite rotation est réalisée suivant un axe X perpendiculaire à l'axe Y.

Les tenseurs axiaux 7 et 8 sont soumis aussi à une tension lorsque l'utilisateur UTL adopte une mauvaise posture. En effet, la mise en tension des tenseurs axiaux 7 et 8 incite, par stimulation, l'utilisateur UTL à réaliser une rotation des épaules vers la face arrière de l'utilisateur UTL telle que représentée sur la figure 5.

En outre, lorsque les tenseurs latéraux 2 et 3 et/ou les tenseurs axiaux 7 et 8 sont soumis à une tension, les moyens d'ouvertures du thorax 6 sont également soumis à une tension car l'assemblage desdits tenseurs 2, 3, 7 et 8 sur les moyens d'ouverture du thorax 6 permet de répartir les forces sur lesdits moyens d'ouverture du thorax 6.

En outre, la zone de stimulation 4 positionnée préférentiellement sur la cinquième vertèbre lombaire permet d'optimiser la stimulation en cas de mauvaise posture. La cinquième vertèbre lombaire est ainsi maintenue dans une position adéquate permettant d'assurer la lordose lombaire naturelle de la colonne vertébrale. En effet, lors d'un mouvement de la colonne vertébrale, la charnière sacro-lombaire C_{SL} (première vertèbre sacrée-cinquième vertèbre lombaire) représentée sur la figure 1, est particulièrement sollicitée. Ainsi, le maintien de la cinquième vertèbre lombaire permet d'assurer un bon positionnement de ladite charnière C_{SL} et ainsi une courbure physiologique. De plus, l'ouverture du thorax dépend du positionnement de la lordose lombaire, le rétablissement d'une lordose lombaire physiologique permet ainsi d'optimiser la respiration. La présence de l'élément 9 fixé sur la zone de stimulation 4 optimise la stimulation de l'utilisateur UTL en cas de mauvaise posture. En effet, une tension sur la zone de stimulation 4 entraîne également une mise en tension de l'élément 9 qui vient en appui sur la cinquième vertèbre lombaire. Ainsi, l'utilisateur UTL ressent une gêne à cause de l'élément 9 ce qui l'amène à se repositionner correctement.

Par ailleurs, une mauvaise posture entraîne une mise en tension de la ceinture 1 qui stimule le bassin de l'utilisateur UTL. Ladite stimulation incite l'utilisateur UTL à adopter un bon positionnement du bassin par rotation selon l'axe X dirigée vers la face arrière de l'utilisateur UTL. Un bon positionnement du bassin permet d'assurer à l'utilisateur UTL une respiration optimale tout en diminuant les contraintes appliquées sur les vertèbres lombaires. On rappelle que les vertèbres lombaires supportent la majeure partie du poids du corps et des contraintes mécaniques, le maintien du bassin permet donc de diminuer ces contraintes. De plus, l'ouverture du thorax dépend aussi du placement du bassin. La ceinture 1 intervient donc en améliorant les mouvements respiratoires et ainsi permettre d'optimiser la respiration de l'utilisateur UTL.

L'utilisateur UTL qui a adopté une mauvaise posture modifie, lorsqu'il porte le dispositif DISP, le positionnement de son dos jusqu'à reprendre une posture correcte c'est-à-dire une posture pour laquelle il ne ressentira plus de stimulations. En effet, lorsque l'utilisateur UTL ne ressent aucune stimulation du dispositif DISP c'est que les éléments 1, 2, 3, 5, 6, 7,8, 9 sont en position d'équilibre soit une position optimale.

En outre, une autre mauvaise posture consiste à avoir les épaules non alignés aboutissant à une asymétrie du corps. Ce mauvais positionnement se traduit par un étirement d'une épaule vers le haut qui étire également la partie du thorax et du bassin correspondantes vers le haut. Dans ce cas, et en considérant que c'est le côté gauche de l'utilisateur UTL qui est étiré vers le haut, une tension dirigée vers le haut est appliquée simultanément sur les éléments 1, 2, 3, 5, 6, 7, 8. Comme explicité précédemment pour le dos voûté, les éléments précités vont appliquer une force dirigée vers le bas de l'utilisateur UTL. Ainsi, l'utilisateur UTL est stimulé sur les zones sur lesquelles une tension est appliquée ce qui l'amène à rétablir un positionnement correcte de son dos.

## Revendications

1. Dispositif (DISP) d'aide pour améliorer la posture d'un utilisateur (UTL) comportant :
▪ une ceinture (1) comprenant une partie avant (101) et une partie arrière (102),
▪ un premier tenseur latéral (2) comprenant une première extrémité (201) et une seconde extrémité (202) et un second tenseur latéral (3) comprenant une première extrémité (301) et une seconde extrémité (302), les premières extrémités (201, 301) du premier tenseur latéral (2) et du second tenseur latéral (3) se rejoignant en une zone de stimulation (4),
▪ une liaison élastique (5) comprenant une première extrémité (501) fixée à la partie arrière (102) de la ceinture (1) et une seconde extrémité (502) fixée à la zone de stimulation (4), ladite liaison élastique (5) étant construite et agencée de sorte que lorsque la partie arrière (102) de la ceinture (1) est positionnée sur les épines iliaques postéro-supérieures de l'utilisateur (UTL), la zone de stimulation (4) est positionnée entre la troisième vertèbre lombaire et la cinquième vertèbre lombaire de l'utilisateur (UTL)
J **caractérisé en ce qu'**il comporte un élément (9) positionné sur la zone de stimulation (4), ledit élément (9) présentant un sommet (91) positionné sur la cinquième vertèbre lombaire L5 lorsque le dispositif (DISP) est porté par l'utilisateur (UTL).

2. Dispositif (DISP) selon la revendication 1 **caractérisé en ce qu'**il comporte des moyens d'ouverture du thorax (6) comprenant une partie avant (601) et une partie arrière (602) fixées à la seconde extrémité (202) du premier tenseur latéral (2) et à la seconde extrémité (302) un second tenseur latéral (3), la jonction entre la partie avant (601) et la partie arrière (602) étant construite et agencé pour se positionner sur les épaules d'un utilisateur (UTL).

3. Dispositif (DISP) selon la revendication 2 **caractérisé en ce que** la seconde extrémité (202) du premier tenseur latéral (2) et la seconde extrémité (302) du second tenseur latéral (3) sont fixées à la partie arrière (602) des moyens d'ouverture du thorax (6), les tenseurs (2, 3) formant chacun un angle compris dans l'intervalle de valeurs [65°, 73°], préférentiellement 71.2° avec un axe sensiblement perpendiculaire à l'axe longitudinal de la ceinture (1) et passant par la zone de stimulation (4).

4. Dispositif (DISP) selon la revendication 2 **caractérisé en ce que** la partie avant (601) et la partie arrière (602) des moyens d'ouverture du thorax (6) forment des bretelles.

5. Dispositif (DISP) selon la revendication 2 **caractérisé en ce que** la partie avant (601) et la partie arrière (602) des moyens d'ouverture du thorax (6) sont réalisées dans un matériau présentant une élasticité plus faible selon un axe vertical que selon un axe horizontal, ledit axe vertical étant sensiblement perpendiculaire à l'axe longitudinal de la ceinture (1).

6. Dispositif (DISP) selon l'une quelconque des revendications 2 à 5 **caractérisé en ce que** les moyens d'ouvertures du thorax (6) comportent un patronage formant des moyens de maintien du thorax, ledit patronage étant ajusté selon une direction sensiblement parallèle à l'axe longitudinal de la ceinture (1).

7. Dispositif (DISP) selon l'une quelconque des revendications 2 à 6 **caractérisé en ce qu'**il comporte :
▪ un premier tenseur axial (7) comprenant une première extrémité (701) fixée à la zone de stimulation (4) et une seconde extrémité (702) fixée aux moyens d'ouverture du thorax (6),
▪ un second tenseur axial (8) comprenant une première extrémité (801) fixée à la zone de stimulation (4) et une seconde extrémité (802) fixée aux moyens d'ouverture du thorax (6).

8. Dispositif (DISP) selon la revendication 7 **caractérisé en ce que** la seconde extrémité (702) du premier tenseur axial (7) et la seconde extrémité (802) du second tenseur axial (8) sont fixées à la partie arrière (602) des moyens d'ouverture du thorax (6), les tenseurs (7, 8) formant chacun un angle compris dans l'intervalle de valeurs [15°, 21°], préférentiellement 18° avec un axe sensiblement perpendiculaire à l'axe longitudinal de la ceinture (1) et passant par la zone de stimulation (4).

## Patentansprüche

1. Hilfs-Vorrichtung (DISP) zum Verbessern der Haltung eines Nutzers (UTL), umfassend:
▪ einen Gürtel (1), umfassend einen vorderen Teil (101) und einen hinteren Teil (102);
▪ einen ersten seitlichen Spanner (2), umfassend ein erstes Ende (201) und ein zweites Ende (202), und einen zweiten seitlichen Spanner (3), umfassend ein erstes Ende (301) und ein zweites Ende (302), wobei die ersten Enden (201, 301) des ersten seitlichen Spanners (2) und des zweiten seitlichen Spanners (3) sich in einem Stimulationsbereich (4) einander annähern,
▪ eine elastische Verbindung (5), umfassend ein erstes Ende (501), das am hinteren Teil (102) des Gürtels (1) befestigt ist, und ein zweites Ende (502), das am Stimulationsbereich (4) befestigt ist, wobei die genannte elastische Verbindung (5) derart aufgebaut und angeordnet ist, dass, wenn der hintere Teil (102) des Gürtels (1) auf der hinteren oberen Beckenwirbelsäule des Benutzers (ULT) positioniert ist, der Stimulationsbereich (4) zwischen dem dritten Lendenwirbel und dem fünften Lendenwirbel des Nutzers (UTL) positioniert ist,
**dadurch gekennzeichnet, dass** sie
ein Element (9) umfasst, das auf dem Stimulationsbereich (4) positioniert ist, wobei das genannte Element (9) eine Spitze (91) aufweist, die auf dem fünften Lendenwirbel L5 positioniert ist, wenn die Vorrichtung (DISP) von dem Nutzer (UTL) getragen ist.

2. Vorrichtung (DISP) gemäß Anspruch 1, **dadurch gekennzeichnet, dass** sie Öffnungsmittel des Thorax (6) umfasst, umfassend einen vorderen Teil (601) und einen hinteren Teil (602), die an dem zweiten Ende (202) des ersten seitlichen Spanners (2) und an dem zweiten Ende (302) eines zweiten seitlichen Spanners (3) befestigt sind, wobei die Nahtstelle zwischen dem vorderen Teil (601) und dem hinteren Teil (602) aufgebaut und angeordnet ist, um sich auf den Schultern eines Nutzers (UTL) zu positionieren.

3. Vorrichtung (DISP) gemäß Anspruch 2, **dadurch gekennzeichnet, dass** das zweite Ende (202) des ersten seitlichen Spanners (2) und das zweite Ende (302) des zweiten seitlichen Spanners (3) am hinteren Teil (602) der Öffnungsmittel des Thorax (6) befestigt sind, wobei die Spanner (2, 3) jeweils einen Winkel bilden, der in dem Werteintervall [65°, 73°], bevorzugt 71,2° inbegriffen ist, mit einer zur Längsachse des Gürtels (1) deutlich lotrechten und durch den Stimulationsbereich (4) hindurchlaufenden Achse.

4. Vorrichtung (DISP) gemäß Anspruch 2, **dadurch gekennzeichnet, dass** der vordere Teil (601) und der hintere Teil (602) der Öffnungsmittel des Thorax (6) Träger bilden.

5. Vorrichtung (DISP) gemäß Anspruch 2, **dadurch gekennzeichnet, dass** der vordere Teil (601) und der hintere Teil (602) der Öffnungsmittel des Thorax (6) aus einem Material realisiert sind, das gemäß einer vertikalen Achse eine geringere Elastizität aufweist als gemäß einer horizontalen Achse, wobei die genannte vertikale Achse deutlich lotrecht zur Längsachse des Gürtels (1) ist.

6. Vorrichtung (DISP) gemäß irgendeinem der Ansprüche 2 bis 5, **dadurch gekennzeichnet, dass** die Öffnungsmittel des Thorax (6) ein Modell umfassen, das Haltemittel des Thorax bildet, wobei das genannte Modell gemäß einer zur Längsachse des Gürtels (1) deutlich parallelen Richtung angepasst ist.

7. Vorrichtung (DISP) gemäß irgendeinem der Ansprüche 2 bis 6, **dadurch gekennzeichnet, dass** sie umfasst:
▪ einen ersten axialen Spanner (7), umfassend ein erstes Ende (701), das an dem Stimulationsbereich (4) befestigt ist, und ein zweites Ende (702), das an den Öffnungsmitteln des Thorax (6) befestigt ist,
▪ einen zweiten axialen Spanner (8), umfassend ein erstes Ende (801), das an dem Stimulationsbereich (4) befestigt ist, und ein zweites Ende (802), das an den Öffnungsmitteln des Thorax (6) befestigt ist.

8. Vorrichtung (DISP) gemäß Anspruch 7, **dadurch gekennzeichnet, dass** das zweite Ende (702) des ersten axialen Spanners (7) und das zweite Ende (802) des zweiten axialen Spanners (8) am hinteren Teil (602) der Öffnungsmittel des Thorax (6) befestigt sind, wobei die Spanner (7, 8) jeweils einen zwischen dem Werteintervall [15°, 21°], bevorzugt 18°, inbegriffenen Winkel mit einer zur Längsachse des Gürtels (1) deutlich lotrechten und durch den Stimulationsbereich (4) hindurchtretenden Achse bildet.

## Claims

1. A device (DISP) for aiding in improving the posture of a user (UTL) including:
▪ a belt (1) comprising a front part (101) and a rear part (102),
▪ a first side tensioner (2) comprising a first end (201) and a second end (202) and a second side tensioner (3) comprising a first end (301) and a second end (302), the first ends (201, 301) of the first side tensioner (2) and second side tensioner (3) joining each other at a stimulation zone (4),
▪ an elastic connection (5) comprising a first end (501) attached to the rear part (102) of the belt (1) and a second end (502) attached to the stimulation zone (4), said elastic connection (5) being built and arranged so that when the rear part (102) of the belt (1) is positioned on the posterior-superior iliac spines of the user (UTL), the stimulation zone (4) is positioned between the third lumbar vertebra and fifth lumbar vertebra of the user (UTL)
**characterised in that** it includes an element (9) positioned on the stimulation zone (4), said element (9) having an apex (91) positioned on the fifth lumbar vertebra L5 when the device (DISP) is worn by the user (UTL).

2. The device (DISP) according to claim 1, **characterised in that** it includes thorax opening means (6) comprising a front part (601) and a rear part (602) attached to the second end (202) of the first side tensioner (2) and to the second end (302) of the second side tensioner (3), the junction between the front part (601) and rear part (602) being built and arranged to be positioned on the shoulders of a user (UTL).

3. The device (DISP) according to claim 2, **characterised in that** the second end (202) of the first side tensioner (2) and the second end (302) of the second side tensioner (3) are attached to the rear part (602) of the thorax opening means (6), the tensioners (2, 3) each forming an angle within the interval of values [65°, 73°], preferentially 71.2° with an axis substantially perpendicular to the longitudinal axis of the belt (1) and zzpassing through the stimulation zone (4).

4. The device (DISP) according to claim 2, **characterised in that** the front part (601) and rear part (602) of the thorax opening means (6) form shoulder straps.

5. The device (DISP) according to claim 2, **characterised in that** the front part (601) and rear part (602) of the thorax opening means (6) are made of a material having a lower elasticity along a vertical axis than along a horizontal axis, said vertical axis being substantially perpendicular to the longitudinal axis of the belt (1).

6. The device (DISP) according to any of claims 2 to 5, **characterised in that** the thorax opening means (6) include a modelling forming means for holding the thorax, said modelling being fitted along a direction substantially parallel to the longitudinal axis of the belt (1).

7. The device (DISP) according to any of claims 2 to 6, **characterised in that** it includes:
▪ a first axial tensioner (7) comprising a first end (701) attached to the stimulation zone (4) and a second end (702) attached to the thorax opening means (6),
▪ a second axial tensioner (8) comprising a first end (801) attached to the stimulation zone (4) and a second end (802) attached to the thorax opening means (6).

8. The device (DISP) according to claim 7, **characterised in that** the second end (702) of the first axial tensioner (7) and the second end (802) of the second axial tensioner (8) are attached to the rear part (602) of the thorax opening means (6), the tensioners (7, 8) each forming an angle within the interval of values [15°, 21°], preferentially 18° with an axis substantially perpendicular to the longitudinal axis of the belt (1) and passing through the stimulation zone (4).
